# EUROPEAN PATENT SPECIFICATION

(11) **EP 1 898 954 B1**
(45) Date of publication and mention of the grant of the patent: **11.08.2010**
(21) Application number: 06754516.0
(22) Date of filing: 22.06.2006
(51) Int. Cl.: A61K 47/22, A61K 9/00

(54) **COMPOSITION AND DOSAGE FORM COMPRISING A SOLID OR SEMI-SOLID MATRIX**
ZUSAMMENSETZUNG UND DOSIERFORM MIT EINER FESTEN ODER HALBFESTEN MATRIX
COMPOSITION ET FORME GALÉNIQUE COMPRENANT UNE MATRICE SOLIDE OU SEMI-SOLIDE

(30) Priority: 05.07.2005 EP 05014555; 05.07.2005 US 595435 P
(43) Date of publication of application: 19.03.2008
(73) Proprietor: Abbott GmbH & Co. KG, 65205 Wiesbaden (DE)
(72) Inventor: ROSENBERG, Jörg, 67158 Ellerstadt (DE); MÄGERLEIN, Markus, 68199 Mannheim (DE); BREITENBACH, Jörg, 68199 Mannheim (DE)
(74) Representative: Reitstötter - Kinzebach
(86) International application number: PCT/EP2006/006026
(87) International publication number: WO 2007/003278

(56) References cited:
- DE-A1- 19 641 436
- US-B1- 6 497 886
- BREITENBACH J ET AL: "CONFOCAL RAMAN-SPECTROSCOPY: ANALYTICAL APPROACH TO SOLID DISPERSIONS AND MAPPING OF DRUGS" PHARMACEUTICAL RESEARCH, NEW YORK, NY, US, vol. 16, no. 7, 1999, pages 1109-1113, XP008015910 ISSN: 0724-8741
- ROSENBERG JOERG ET AL: "Melt extrusion: Assessing the potential of poorly soluble drugs to form glass solutions." ABSTRACTS OF PAPERS AMERICAN CHEMICAL SOCIETY, vol. 226, no. 1-2, 2003, page PMSE 128, XP009054930 & 226TH ACS (AMERICAN CHEMICAL SOCIETY) NATIONAL MEETING; NEW YORK, NY, USA; SEPTEMBER 07-11, 2003 ISSN: 0065-7727

## Description

The present invention relates to a composition comprising a solid or semi-solid matrix having at least one active ingredient uniformly dispersed therein, a pharmaceutical dosage form comprising the matrix, as well as to a process for their preparation.

A measure of the potential usefulness of an oral dosage form of a pharmaceutical agent is the bioavailability observed following oral administration of said dosage form. Various factors can affect the bioavailability of a drug when administered orally. These factors include aqueous solubility, drug absorption throughout the gastrointestinal tract, dosage strength and first pass effect. Aqueous solubility is one of the most important of these factors. Unfortunately, many active ingredients are typically characterized by poor aqueous solubility.

For a variety of reasons, such as patient compliance and taste-masking, a solid dosage form is usually preferred over a liquid dosage form. In most instances, however, solid oral dosage forms of a drug provide a lower bioavailability than oral solutions of the drug.

There have been attempts to improve the bioavailability provided by solid dosage forms by forming solid dispersions or solid solutions of the drug. Solid solutions are preferred physical systems because the components therein readily form liquid solutions when brought into contact with a liquid medium such as gastric juice. This increased propensity for dissolution may be attributed at least in part to the fact that the energy required for dissolving the components from a solid solution is less than that required for dissolving the components from a crystalline or microcrystalline solid phase.

A continuous process for producing solid pharmaceutical forms, including solid solution products, has been known for some time and entails converting a melt of polymeric binder which contains active ingredients and is free from solvents into the required drug form by injection molding or extrusion and subsequent shaping (see, for example, EP-A-240 904, EP-A-240 906 and EP-A-337 256). Satisfactory results are obtained in this process when the active ingredient has a low melting point and/or a high solubility in the molten polymeric binder. Active ingredients having a low melting point are liquefied upon contact with the polymeric binder melt, and the liquified active ingredient can be readily dispersed in the polymeric binder melt. Alternatively, active ingredients having a high solubility in the molten polymeric binder readily dissolve in the polymeric binder melt.

Problems occur when the active ingredient has a high melting point and/or a limited solubility in the molten polymeric binder. Adequate dispersion of the active ingredient may require high temperatures, a relatively long mixing time and/or high shear in order to bring about sufficient mixing of the active ingredient with the polymeric binder melt. This may result in local overheating and damage to the product, especially when a shear- and temperature-sensitive active ingredient is being used.

WO 98/10752 discloses a process for producing solid dosage forms in which a polymeric binder, optionally an active ingredient, and additives are mixed and extruded. The reference suggests dissolving temperature-sensitive active ingredients in a solvent and introducing the solvent solution into the extruder. The process requires that the solvent be removed from the melt by evaporation in the extruder.

WO 98/15291 relates to the use of 1,3-bis(pyrrolidon-1-yl)-butane and other 1,3-bis-(lactamyl)-propanes as a solvent in pharmaceutical and cosmetic agents.

Breitenbach, J. et al. in Pharmaceutical Research, Vol. 16, No. 7, 1999 p. 1109-1113 compare the state of ibuprofen in 1,3-bis(pyrrolidon-1-yl)-butane with a polymeric matrix of polyvinylpyrrolidon.

DE 196 41 436 discloses complexes of hydrogen peroxide and 1,3-bis(N-lactamyl)propans.

There is a continuing need for the development of oral solid dosage forms which have suitable oral bioavailability and stability and which are adapted to active ingredients having a high melting point and/or a limited solubility in molten polymeric binders.

It is known that 1,3-bis(lactamyl)-butanes have a high dissolution ability for many active ingredients. It has now been discovered, quite surprisingly, that considerable amounts of 1,3-bis(lactamyl)-butanes can be incorporated into the matrix of a solid dosage form without compromising the mechanical and storage properties of said dosage form. Thus the invention allows for the incorporation of active ingredients in a liquid dissolved state into a solid or semi-solid matrix and dispenses with the requirement of removing the solvent.

The invention provides a composition which comprises a solid or semi-solid matrix having at least one active ingredient uniformly dispersed therein, the matrix comprising at least one pharmaceutically acceptable matrix-forming agent selected from pharmaceutically acceptable polymers having a glass transition temperature Tg of from 50 ° to 180 °C; and 1,3-bis(pyrrolidon-1-yl)-butane, wherein the matrix contains less than 6 % by weight of water or any compound that is liquid at ambient temperature and has a higher volatility than water, and wherein the solubility of the active ingredient in water at 25 °C is less than 1g/100 ml.

The composition according to the invention comprises a solid or semi-solid matrix having at least one active ingredient uniformly dispersed therein. The term "semi-solid matrix" is intended to mean a non-flowable system that may be deformed when acted upon by a force. A "solid matrix" is typically brittle and breaks into pieces when a deforming force is applied thereto.

The invention also provides a dosage form, preferably a solid dosage form, comprising (or consisting of) the above composition.

For easier handling, a semi-solid matrix may require an outer casing that completely envelops the semi-solid matrix, such as a coating or capsule shell, e. g. a soft or hard gelatine capsule shell.

On the other hand, a solid matrix may be shaped into a desired form and used as a dosage form *as such.* Alternatively, the solid matrix may be ground and compressed into a tablet. Alternatively, the solid matrix may be ground and the ground product may be filled into a capsule.

One or more active ingredients are dispersed evenly throughout the matrix. This encompasses systems having small particles, typically of less than 1 µm in diameter, of active ingredient in the matrix phase. These systems do not contain any significant amounts of active ingredients in their crystalline or microcrystalline state, as evidenced by thermal analysis (DSC) or X-ray diffraction analysis (WAXS). Typically, at least 98 % by weight of the total amount of active ingredients is present in an amorphous state.

When said dispersion of the components is such that the system is chemically and physically uniform or homogenous throughout or consists of one phase (as defined in thermodynamics), such a dispersion is called a "solid solution". Solid solutions of active ingredients are preferred physical systems.

The compound 1,3-bis(pyrrolidon-1-yl)-butane acts as a non-volatile solvent for the active ingredient and, optionally, further ingredients. Preferably, the matrix is essentially free of non-volatile solvents (solvents having a lower volatility than water) other than the compound. Usually, the matrix contains less than 6 %, preferably less than 3 %, and most preferred less than 2 % by weight of a non-volatile solvent other than the compound.

The matrix does not contain significant amounts of volatile solvents. The term "volatile solvent" is intended to encompass water and any compound that is liquid at ambient temperature and has a higher volatility than water. The matrix contains less than 6 %, preferably less than 3 %, and most preferred less than 2 % by weight of a volatile solvent.

Relative to the total weight of active ingredient(s) and other components constituting the matrix, preferred compositions of the invention comprise a matrix comprising:
from about 1 to 30 % by weight (preferably 5 to 25 % by weight) of 1,3-bis(pyrrolidon-1-yl)-butane,
   from about 50 to 98 % by weight (preferably 60 to 90 % by weight) of the matrix-forming agent (or any combination of such matrix-forming agents),
   from about 1 to 49 % by weight (preferably 1 to 30 % by weight) of an active ingredient or a combination of active ingredients,
   from 0 to 25 % by weight (preferably 1 to 15 % by weight) of additives.

The preparation of the 1,3-bis(pyrrolidon-1-yl)-butane is described in detail in WO 98/15291.

The matrix-forming agent is capable of setting or gelling from a liquified state, e. g. from a molten or dissolved state, to form a continuous matrix. Where a solid matrix is desired, the matrix-forming agent is selected so as to form a continuous matrix of sufficient mechanical stability. Mixtures of matrix-forming agents can, of course, be used. The matrix-forming agent is a pharmaceutically acceptable polymer or a mixture of pharmaceutically acceptable polymers. Usually, pharmaceutically acceptable polymers are water-soluble or at least water-dispersible.

The pharmaceutically acceptable polymer employed in the invention has a Tg of from 50 ° to 180 °C. "Tg" means glass transition temperature. Methods for determining Tg values of organic polymers are described in "Introduction to Physical Polymer Science", 2nd Edition by L.H. Sperling, published by John Wiley & Sons, Inc., 1992. The Tg value can be calculated as the weighted sum of the Tg values for homopolymers derived from each of the individual monomers i that make up the polymer, i.e. Tg = E Wᵢ Xᵢ where W is the weight percent of monomer i in the organic polymer and X is the Tg value for the homopolymer derived from monomer i. Tg values for the homopolymers are indicated in "Polymer Handbook", 2nd Edition by J. Brandrup and E.H. Immergut, Editors, published by John Wiley & Sons, Inc., 1975.

Pharmaceutically acceptable polymers having a Tg as defined above allow the preparation of solid dispersions that are mechanically stable and, within ordinary temperature ranges, sufficiently temperature stable so that said solid dispersions may be used as dosage forms without further processing or can be compacted to tablets with only a small amount of tabletting aids.

The pharmaceutically acceptable polymer comprised in the composition is a polymer that preferably has an apparent viscosity, when dissolved at 20 °C in an aqueous solution at 2 % (w/v) of 1 to 50 000 mPa.s more preferably of 1 to 10 000 mPa.s, and most preferably of 5 to 100 mPa.s. For example, preferred pharmaceutically acceptable polymers can be selected from the group comprising:
homopolymers of N-vinyl lactams, especially polyvinylpyrrolidone (PVP),
copolymers of a N-vinyl lactam and and one or more comonomers copolymerizable therewith, the comonomers being selected from nitrogen-containing monomers and oxygen-containing monomers; especially a copolymer of N-vinyl pyrrolidone and a vinyl carboxylate, preferred examples being a copolymer of N-vinyl pyrrolidone and vinyl acetate or a copolymer of N-vinyl pyrrolidone and vinyl propionate;
cellulose esters and cellulose ethers, in particular methylcellulose and ethylcellulose, hydroxyalkylcelluloses, in particular hydroxypropylcellulose, hydroxyalkylalkylcelluloses, in particular hydroxypropylmethylcellulose, cellulose phthalates or succinates, in particular cellulose acetate phthalate and hydroxypropylmethylcellulose phthalate, hydroxypropylmethylcellulose succinate or hydroxypropylmethylcellulose acetate succinate;
polyvinyl alcohol-polyethylene glycol-graft copolymers (available as Kollicoat® IR from BASF AG, Ludwigshafen, Germany);
high molecular polyalkylene oxides such as polyethylene oxide and polypropylene oxide and copolymers of ethylene oxide and propylene oxide;
polyacrylates and polymethacrylates such as methacrylic acid/ethyl acrylate copolymers, methacrylic acid/methyl methacrylate copolymers, butyl methacrylate/2-dimethylaminoethyl methacrylate copolymers, poly(hydroxyalkyl acrylates) and poly(hydroxyalkyl methacrylates);
polyacrylamides;
vinyl acetate polymers such as copolymers of vinyl acetate and crotonic acid, partially hydrolyzed polyvinyl acetate (also referred to as partially saponified "polyvinyl alcohol");
polyvinyl alcohol;
poly(hydroxy acids) such as poly(lactic acid), poly(glycolic acid), poly(3-hydroxybutyrate) and poly(3-hydroxybutyrate-co-3-hydroxyvalerate);
or mixtures of one or more thereof.

Among these, homopolymers or copolymers of N-vinyl pyrrolidone, in particular a copolymer of N-vinyl pyrrolidone and vinyl acetate, are preferred. A particularly preferred polymer is a copolymer of 60 % by weight of the copolymer N-vinyl pyrrolidone and 40 % by weight of the copolymer vinyl acetate.

Hydroxypropylcellulose is another example of a particularly preferred polymer.

Active ingredients used to carry out the present invention are biologically active agents and include those which exert a local physiological effect, as well as those which exert a systemic effect, after oral administration. The invention is useful for water-insoluble or poorly water-soluble (or "lipophilic") compounds. Compounds are considered water-insoluble or poorly water-soluble when their solubility in water at 25 °C is less than 1 g/100 ml.

Solid dispersion products are typically prepared by a process known as melt extrusion. In order to obtain a homogeneous distribution and a sufficient degree of dispersion of the active ingredient, an active ingredient-containing melt is kept in the heated barrel of a melt extruder during a sufficient residence time. As the manufacture of the compost-. - tions according to the invention uses a preformed active ingredient solution, the residence time of the active ingredient in the extruder can be significantly reduced. Thus, the invention is particularly adapted for the formulation of active ingredients that are susceptible to thermal decomposition.

The invention greatly reduces the necessity of handling active ingredient powders which are prone to dust formation. Dust exposure may constitute significant health hazards to the personnel of a manufacturing plant, in particular where high potent ingredients are concerned. American Industrial Hygiene Association Journal 57:3342 (1996) assigns actives into performance-based exposure control limit (PB-ECL) categories, based on the degree to which exposure impacts human health. The PB-ECL categories range from PB-ECL 1 (low severity of acute effects; no chronic effects) to PB-ECL 5 (high severity of acute effects; severe chronic effects). The invention is particularly useful for active ingredients that fall within PB-ECL categories 3, 4, or 5, and especially those falling .within PB-ECL categories 4 or 5.

Examples of suitable active substances include, but are not limited to:
analgesic and anti-inflammatory drugs such as NSAIDs, fentanyl, indomethacin, ibuprofen, ketoprofen, nabumetone, paracetamol, piroxicam, meloxicam, tramadol, and COX-2 inhibitors such as celecoxib and rofecoxib;
anti-arrhythmic drugs such as procainamide, quinidine and verapamil;
antibacterial and antiprotozoal agents such as amoxicillin, ampicillin, benzathine penicillin, benzylpenicillin, cefaclor, cefadroxil, cefprozil, cefuroxime axetil, cephalexin, chloramphenicol, chloroquine, ciprofloxacin, clarithromycin, clavulanic acid, clindamycin, doxyxycline, erythromycin, flucloxacillin sodium, halofantrine, isoniazid, kanamycin sulphate, lincomycin, mefloquine, minocycline, nafcillin sodium, nalidixic acid, neomycin, nortloxacin, ofloxacin, oxacillin, phenoxymethyl-penicillin potassium, pyrimethamine-sulfadoxime and streptomycin;
anti-coagulants such as warfarin;
antidepressants such as amitriptyline, amoxapine, butriptyline, clomipramine, desipramine, dothiepin, doxepin, fluoxetine, reboxetine, amineptine, selegiline, gepirone, imipramine, lithium carbonate, mianserin, milnacipran, nortriptyline, paroxetine, sertraline and 3-[2-[3,4-dihydrobenzofuro[3,2-c]pyridin-2(1H)-yl]ethyl]-2-methyl-4H-pyrido[1,2-a]pyrimidin-4-one;
anti-diabetic drugs such as glibenclamide and metformin;
anti-epileptic drugs such as carbamazepine, clonazepam, ethosuximide, gabapentin, lamotrigine, levetiracetam, phenobarbitone, phenytoin, primidone, tiagabine, topiramate, valpromide and vigabatrin;
antifungal agents such as amphotericin, clotrimazole, econazole, fluconazole, flucytosine, griseofulvin, itraconazole, ketoconazole, miconazole nitrate, nystatin, terbinafine and voriconazole;
antihistamines such as astemizole, cinnarizine, cyproheptadine, decarboethoxyloratadine, fexofenadine, flunarizine, levocabastine, loratadine, norastemizole, oxatomide, promethazine and terfenadine;
anti-hypertensive drugs such as captopril, enalapril, ketanserin, lisinopril, minoxidil, prazosin, ramipril, reserpine, terazosin and telmisartan;
anti-muscarinic agents such as atropine sulphate and hyoscine;
antineoplastic agents and antimetabolites such as platinum compounds, such as cis-platin and carboplatin; taxanes such as paclitaxel and docetaxel; tecans such as camptothecin, irinotecan and topotecan; vinca alkaloids such as vinblastine, vindecine, vin-cristine and vinorelbine; nucleoside derivatives and folic acid antagonists such as 5-fluorouracil, capecitabine, gemcitabine, mercaptopurine, thioguanine, cladribine and methotrexate; alkylating agents such as the nitrogen mustards, e.g. cyclophosphamide, chlorambucil, chiormethine, iphosphamide, melphalan, or the nitrosoureas, e.g. carmustine, lomustine, or other alkylating agents, e.g. busulphan, dacarbazine, procarbazine, thiotepa; antibiotics such as daunorubicin, doxorubicin, idarubicin, epirubicin, bleomycin, dactinomycin and mitomycin; HER 2 antibody such as trastuzumab; podophyllotoxin derivatives such as etoposide and teniposide; famesyl transferase inhibitors; anthrachinon derivatives such as mitoxantron;
anti-migraine drugs such as alniditan, naratriptan and sumatriptan;
anti-Parkinsonian drugs such as bromocryptine mesylate, levodopa and selegiline;
antipsychotic, hypnotic and sedating agents such as alprazolam, buspirone, chlordiazepoxide, chlorpromazine, clozapine, diazepam, flupenthixol, fluphenazine, flurazepam, 9-hydroxyrisperidone, lorazepam, mazapertine, olanzapine, oxazepam, pimozide, pipamperone, piracetam, promazine, risperidone, selfotel, seroquel, sertindole, sulpiride, temazepam, thiothixene, triazolam, trifluperidol, ziprasidone and zolpidem;
anti-stroke agents such as lubeluzole, lubeluzole oxide, riluzole, aptiganel, eliprodil and remacemide;
antitussives such as dextromethorphan and laevodropropizine;
antivirals such as acyclovir, ganciclovir, loviride, tivirapine, zidovudine, lamivudine, zidovudine/lamivudine, didanosine, zalcitabine, stavudine, abacavir, lopinavir, amprenavir, nevirapine, efavirenz, delavirdine, indinavir, nelfinavir, ritonavir, saquinavir, adefovir and hydroxyurea;
beta-adrenoceptor blocking agents such as atenolol, carvedilol, metoprolol, nebivolol and propanolol;
cardiac inotropic agents such as amrinone, digitoxin, digoxin and milrinone;
corticosteroids such as beclomethasone dipropionate, betamethasone, budesonide, dexamethasone, hydrocortisone, methylprednisolone, prednisolone, prednisone and triamcinolone;
disinfectants such as chlorhexidine;
diuretics such as acetazolamide, furosemide, hydrochlorothiazide and isosorbide;
enzymes;
essential oils such as anethole, anise oil, caraway, cardamom, cassia oil, cineole, cinnamon oil, clove oil, coriander oil, dementholised mint oil, dill oil, eucalyptus oil, eugenol, ginger, lemon oil, mustard oil, neroli oil, nutmeg oil, orange oil, peppermint, sage, spearmint, terpineol and thyme;
gastro-intestinal agents such as cimetidine, cisapride, clebopride, diphenoxylate, domperidone, famotidine, lansoprazole, loperamide, loperamide oxide, mesalazine, metoclopramide, mosapride, nizatidine, norcisapride, olsalazine, omeprazole, pantoprazole, perprazole, prucalopride, rabeprazole, ranitidine, ridogrel and sulphasalazine;
haemostatics such as aminocaproic acid;
lipid regulating agents such as atorvastatin, fenofibrate, fenofibric acid, lovastatin, pravastatin, probucol and simvastatin;
local anaesthetics such as benzocaine and lignocaine;
opioid analgesics such as buprenorphine, codeine, dextromoramide, dihydrocodeine, hydrocodone, oxycodone and morphine;
parasympathomimetics and anti-dementia drugs such as AIT-082, eptastigmine, galanthamine, metrifonate, milameline, neostigmine, physostigmine, tacrine, donepezil, rivastigmine, sabcomeline, talsaclidine, xanomeline, memantine and lazabemide;
peptides and proteins such as antibodies, becaplermin, cyclosporine, tacrolimus, erythropoietin, immunoglobulins and insuline;
sex hormones such as oestrogens: conjugated oestrogens, ethinyloestradiol, mestranol, oestradiol, oestriol, oestrone; progestogens; chlormadinone acetate, cyproterone acetate, 17-deacetyl norgestimate, desogestrel, dienogest, dydrogesterone, ethynodiol diacetate, gestodene, 3-keto desogestrel, levonorgestrel, lynestrenol, medroxy-progesterone acetate, megestrol, norethindrone, norethindrone acetate, norethisterone, norethisterone acetate, norethynodrel, norgestimate, norgestrel, norgestrienone, progesterone and quingestanol acetate;
stimulating agents such as sildenafil;
vasodilators such as amlodipine, buflomedil, amyl nitrite, diltiazem, dipyridamole, glyceryl trinitrate, isosorbide dinitrate, lidoflazine, molsidomine, nicardipine, nifedipine, oxpentifylline and pentaerythritol tetranitrate;
their N-oxides, their pharmaceutically acceptable acid or base addition salts and their stereochemically isomeric forms.
Pharmaceutically acceptable acid addition salts comprise the acid addition salt forms which can be obtained conveniently by treating the base form of the active ingredient with appropriate organic and anorganic acids.

Active ingredients containing an acidic proton may be converted into their non-toxic metal or amine addition salt forms by treatment with appropriate organic and inorganic bases.

The term addition salt also comprises the hydrates and solvent addition forms which the active ingredients are able to form. Examples of such forms are, for example, hydrates, alcoholates and the like.

The N-oxide forms of the active ingredients comprise those active ingredients in which one or several nitrogen atoms are oxidized to the so-called N-oxide.

The term "stereochemically isomeric forms" defines all possible stereoisomeric forms which the active ingredients may possess. In particular, stereogenic centers may have the R- or S-configuration and active ingredients containing one or more double bonds may have the E- or Z-configuration.

The invention is particularly adapted for the manufacture of dosage forms incorporating active ingredients having relatively high melting points, e.g. active ingredients having a melting point of 170 °C or higher. Typical examples thereof include meloxicam and telmisartan.

The matrix of the dosage form may comprise one or more additives selected from pharmaceutically acceptable surfactants, flow regulators, disintegrants, bulking agents and lubricants.

The term "pharmaceutically acceptable surfactant" as used herein refers to a pharmaceutically acceptable non-ionic surfactant. Incorporation of surfactants is especially preferred for matrices containing poorly water-soluble active ingredients. The surfactant may effectuate an instantaneous emusification of the active ingredient released from the dosage form and prevent precipitation of the active ingredient in the aqueous fluids of the gastrointestinal tract.

Preferred surfactants are selected from:
polyoxyethylene alkyl ethers, e.g. polyoxyethylene (3) lauryl ether, polyoxyethylene (5) cetyl ether, polyoxyethylene (2) stearyl ether, polyoxyethylene (5) stearyl ether; polyoxyethylene alkylaryl ethers, e.g. polyoxyethylene (2) nonylphenyl ether, polyoxyethylene (3) nonylphenyl ether, polyoxyethylene (4) nonylphenyl ether or polyoxyethylene (3) octylphenyl ether;
polyethylene glycol fatty acid esters, e.g. PEG-200 monolaurate, PEG-200 dilaurate, PEG-300 dilaurate, PEG-400 dilaurate, PEG-300 distearate or PEG-300 dioleate;
alkylene glycol fatty acid mono esters, e.g. propylene glycol monolaurate (Lauroglycol®);
sucrose fatty acid esters, e.g. sucrose monostearate, sucrose distearate, sucrose monolaurate or sucrose dilaurate;
sorbitan fatty acid mono esters such as sorbitan mono laurate (Span® 20), sorbitan monooleate, sorbitan monopalmitate (Span® 40), or sorbitan stearate,
polyoxyethylene castor oil derivates, e.g. polyoxyethyleneglycerol triricinoleate or polyoxyl 35 castor oil (Cremophor® EL; BASF Corp.) or polyoxyethyleneglycerol oxystearate such as polyethylenglycol 40 hydrogenated castor oil (Cremophor® RH 40) or polyethylenglycol 60 hydrogenated castor oil (Cremophor® RH 60); or
block copolymers of ethylene oxide and propylene oxide, also known as polyoxyethylene polyoxypropylene block copolymers or polyoxyethylene polypropyleneglycol such as Poloxamer® 124, Poloxamer® 188, Poloxamer® 237, Poloxamer® 388, or Poloxamer® 407 (BASF Wyandotte Corp.); or
mono fatty acid esters of polyoxyethylene (20) sorbitan, e.g. polyoxyethylene (20) sorbitan monooleate (Tween® 80), polyoxyethylene (20) sorbitan monostearate (Tween® 60), polyoxyethylene (20) sorbitan monopalmitate (Tween® 40), polyoxyethylene (20) sorbitan monolaurate (Tween® 20), or mixtures of one or more thereof.

The dosage forms of the invention may contain at least one conventional additive such as flow regulators, lubricants, bulking agents (fillers) and disintegrants.

Various methods can be used for manufacturing the solid dosage forms according to the invention. These methods usually involve forming a solution of an active ingredient in a sufficient amount of 1,3-bis(pyrrolidon-1-yl)-butane to obtain an active ingredient solution. In preferred embodiments, a preformed active ingredient solution (that may contain part or all of the optional ingredients) is combined with the matrix-forming agent and the mixture is heated to obtain a melt. Alternatively, the active ingredient solution is formed *in situ* by combining the active ingredient in a solid state, e. g. crystalline state, a sufficient amount of the1,3-bis(pyrrolidon-1-yl)-butane, the matrix-forming agent and any optional ingredients and heating the thus obtained mixture to obtain a homogeneous melt. It is believed that during the mixing and heating operations the active ingredient becomes dissolved in the 1,3-bis(pyrrolidon-1-yl)-butane; thus the presence of 1,3-bis(pyrrolidon-1-yl)-butane assists in the homogeneous distribution of the active ingredient in the matrix provided by the matrix-forming agent.

In order to prepare a preformed active ingredient solution, the active ingredient is brought into contact with 1,3-bis(pyrrolidon-1-yl)-butane, usually with agitation. If desired, the solution may be heated to accelerate dissolution or to enhance the solubility of the active ingredient. Conveniently, the temperature is in the range of from about 20 °C to about 150 °C, preferably from about 20 °C to about 100 °C; higher temperatures are usually not advisable. Typically, the active ingredient content of the solution is below the value at which the solution is saturated. However, the active ingredient solution may as well be saturated or even supersaturated, i. e., the active ingredient solution may contain suspended or undissolved active ingredient.

The dosage forms according to the invention are preferably obtained by a method which comprises:
a) dissolving the active ingredient in a sufficient amount of 1,3-bis(pyrrolidon-1-yl)-butane to obtain an active ingredient solution;
b) providing a powdery composition comprising the matrix-forming agent;
c) blending the active ingredient solution into the powdery composition to obtain a uniform granulate;
d) heating the uniform granulate to an elevated temperature to obtain a melt; and
e) allowing the melt to solidify to obtain a solid dispersion product.

Alternatively, the dosage forms according to the invention are obtained by a method which comprises:
a) heating the matrix-forming agent to an elevated temperature to obtain a matrix-forming agent melt,
b) dissolving the active ingredient in a sufficient amount of 1,3-bis(pyrrolidon-1-yl)-butane to obtain an active ingredient solution,
c) adding the active ingredient solution to the matrix-forming agent melt, and
d) allowing the melt to solidify to obtain a solid dispersion product.

Evidently, two or more active ingredients can be incorporated by blending a first active ingredient (or a first active ingredient combination) into the powdery matrix-forming agent composition, heating, and adding a solution of a second active ingredient (or a second active ingredient combination) to the melt.

"Melting" means a transition into a liquid or rubbery state in which it is possible for one component to be homogeneously embedded in the other. Melting usually involves heating above the softening point of the matrix-forming agent such as a pharmaceutically acceptable polymer. The preparation of the melt can take place in a variety of ways.

Usually, the melt temperature is in the range of 70 to 250 °C, preferably 80 to 180 °C, most preferably 100 to 140 °C.

The active ingredients are employed as a solution in a suitable amount of 1,3-bis(pyrrolidon-1-yl)-butane. This solution is mixed with and into the matrix-forming agent either before or after the melting of said matrix-forming agent. Usually, the melt is homogenized in order to disperse the active ingredient solution efficiently.

Various additives may be included in the melt, for example flow regulators such as colloidal silica; lubricants, fillers, disintegrants, plasticizers, stabilizers such as antioxidants, light stabilizers, radical scavengers or stabilizers against microbial attack.

The melting and/or mixing takes place in an apparatus customarily used for this purpose. Particularly suitable are extruders or kneaders. Suitable extruders include single screw extruders, intermeshing screw extruders or else multiscrew extruders, preferably twin screw extruders, which can be corotating or counterrotating and are optionally equipped with kneading disks. It will be appreciated that the working temperatures will also be determined by the kind of extruder or the kind of configuration within the extruder that is used. Part of the energy needed to melt, mix and dissolve the components in the extruder can be provided by heating elements. However, the friction and shearing of the material in the extruder may also provide the mixture with a substantial amount of energy and aid in the formation of a homogeneous melt of the components.

The melt ranges from pasty to viscous. Before allowing the melt to solidify, the melt may be shaped into virtually any desired shape. Shaping of the extrudate is conveniently carried out by a calender with two counter-rotating rollers with mutually matching depressions on their surface. A broad range of tablet forms can be attained by using rollers with different forms of depressions. Alternatively, the extrudate is cut into pieces, either before (hot-cut) or after solidification (cold-cut).

In a preferred embodiment the melt is extruded through a slot die to obtain a film. The film thus obtained is optionally stretched, axially or biaxially. The film can be cut into the desired size.

Optionally, the resulting solid dispersion product is milled or ground to granules. The granules may then be compacted. Compacting means a process whereby a powder mass comprising the granules is condensed under high pressure in order to obtain a compact with low porosity, e.g. a tablet. Compression of the powder mass is usually done in a tablet press, more specifically in a steel die between two moving punches.

Preferably, at least one additive selected from flow regulators, disintegrants, bulking agents (fillers) and lubricants is used in compacting the granules. Disintegrants promote a rapid disintegration of the compact in the stomach and keep the granules which are liberated separate from one another. Suitable disintegrants are crosslinked polymers such as crosslinked polyvinyl pyrrolidone and crosslinked sodium carboxymethylcellulose. Suitable bulking agents (also referred to as "fillers") are selected from lactose, calcium hydrogenphosphate, microcrystalline cellulose (Avicel®), silicates, in particular silicium dioxide, talc, potato or corn starch, and isomalt.

Suitable flow regulators are selected from highly dispersed silica (Aerosil®), and animal or vegetable fats or waxes.

A lubricant is preferably used in compacting the granules. Suitable lubricants are selected from polyethylene glycol (e.g., having a Mw of from 1000 to 6000), magnesium and calcium stearates, sodium stearyl fumarate, and the like.

Various other additives may be used, for example dyes such as azo dyes, organic or inorganic pigments such as iron oxides or titanium dioxide, or dyes of natural origin; stabilizers such as antioxidants, light stabilizers, radical scavengers and stabilizers against microbial attack.

Dosage forms according to the invention may be provided as dosage forms consisting of several layers, for example laminated or multilayer tablets. They can be in open or closed form. "Closed dosage forms" are those in which one layer is completely surrounded by at least one other layer. Multilayer forms have the advantage that two active ingredients which are incompatible with one another can be processed and that the release characteristics of the active ingredient(s) can be controlled. For example, it is possible to provide an initial dose by including an active ingredient in one of the outer layers, and a maintenance dose by including the active ingredient in the inner layer(s). Multilayer tablets types may be produced by compressing two or more layers of granules. Alternatively, multilayer dosage forms may be produced by a process known as "coextrusion". In essence, the process comprises the preparation of at least two different melt compositions as explained above, and the passing of these molten compositions into a joint coextrusion die. The shape of the coextrusion die depends on the required drug form. For example, dies with a plain die gap, called slot dies, and dies with an annular slit are suitable.

In order to faciliate the intake of such a dosage form by a mammal, it is advantageous to give the dosage form an appropriate shape. Large tablets that can be swallowed comfortably are therefore preferably elongated rather than round in shape.

A film coat on the tablet further contributes to the ease with which it can be swallowed. A film coat also improves taste and provides an elegant appearance. If desired, the film coat may be an enteric coat. The film coat usually includes a polymeric film-forming material such as hydroxypropyl methylcellulose, hydroxypropylcellulose, and acrylate or methacrylate copolymers. Besides a film-forming polymer, the film-coat may further comprise a plasticizer, e.g. polyethylene glycol, a surfactant, e.g. a Tween® type, and optionally a pigment, e.g. titanium dioxide or iron oxides. The film-coating may also comprise talc as an anti-adhesive. The film coat usually accounts for less than about 5 % by weight of the dosage form.

The following examples will serve to further illustrate the invention without limiting it. In examples 1 to 8, a dye (Sudan Red III) was used as a model compound in order to visualize the distribution of the the compound in the solid dispersion product. It will be appreciated that biologically active ingredients can be processed in an essentially similar manner.

### Example 1

Sudan Red III was dissolved in 1,3-bis(pyrrolidon-1-yl)-butane at a concentration of 0.5 % by weight. 5 parts by weight of the resulting deep red solution were blended with 95 parts by weight of Kollidon VA 64 (N-vinyl pyrrolidone/vinyl acetate copolymer 60:40). The mixture was granulated in a lab-scale high-shear mixer. The granulate thus obtained was fed into a corotating twin-screw extruder and was extruded at a temperature of 130 °C. The extrudate solidified into a homogeneous clear red, brittle, non-tacky mass. The glass transition temperature of the extrudate as determined by DSC was 86 °C.

### Example 2

Example 1 was repeated, except that 10 parts by weight of Sudan Red solution and 90 parts by weight of Kollidon VA 64 were used. Again, a homogeneous clear red, brittle, non-tacky mass was obtained. The glass transition temperature of the extrudate as determined by DSC was 76 °C

### Example 3

Example 1 was repeated, except that 15 parts by weight of Sudan Red solution and 85 parts by weight of Kollidon VA 64 were used. Again, a homogeneous clear red, brittle, non-tacky mass was obtained. The glass transition temperature of the extrudate as determined by DSC was 58 °C

### Example 4

Example 1 was repeated, except that 20 parts by weight of Sudan Red solution and 80 parts by weight of Kollidon VA 64 were used. Again, a homogeneous clear red, brittle, non-tacky mass was obtained. The glass transition temperature of the extrudate as determined by DSC was 49 °C.

### Example 5

Example 1 was repeated, except that 30 parts by weight of Sudan Red solution and 70 parts by weight of Kollidon VA 64 were used. Melt extrusion was carried out at a temperature of 110 °C. A homogeneous clear red, brittle, slightly tacky mass was obtained. The glass transition temperature of the extrudate as determined by DSC was 24 °C.

### Example 6

Sudan Red III was diissolved in 1,3-bis(pyrrolidon-1-yl)-butane at a concentration of 0.5 % by weight. 20 parts by weight of the resulting deep red solution were blended with 80 parts by weight of Kollidon 25 (N-vinyl pyrrolidone homopolymer). The mixture was granulated in a lab-scale high-shear mixer. The granulate thus obtained was fed into a corotating twin-screw extruder and was extruded at a temperature of 110 °C. The extrudate solidified into a homogeneous clear red, brittle, non-tacky mass. The glass transition temperature of the extrudate as determined by DSC was 74 °C.

### Example 7

Sudan Red III was dissolved in 1,3-bis(pyrrolidon-1-yl)-butane at a concentration of 0.5 % by weight. 10 parts by weight of the resulting deep red solution were blended with 90 parts by weight of Klucel EF (hydroxypropyl cellulose). The mixture was granulated in a lab-scale high-shear mixer. The granulate thus obtained was fed into a corotating twin-screw extruder and was extruded at a temperature of 110 °C. The extrudate solidified into a homogeneous clear red, resilient, non-tacky mass. The glass transition temperature of the extrudate as determined by DSC was 1°C.

### Example 8

Example 7 was repeated, except that 20 parts by weight of Sudan Red solution and 80 parts by weight of Klucel EF were used. Melt extrusion was carried out at a temperature of 100 °C. A homogeneous clear red, resilient, slightly tacky mass was obtained. The glass transition temperature of the extrudate as determined by DSC was -10 °C.

### Example 9

Tramadol hydrochloride (2,5 g) was dissolved in 1,3-bis(pyrrolidon-1-yl)-butane (7,5 g) with gentle heating. 5 g of the resulting solution were mixed with 25 g Kollidon VA 64 in a laboratory mill (IKA blade mill) to obtain a homogeneous granulate. The composition of the granulate was: 4,2 % by weight of tramadol hydrochloride, 12,5 % by weight of 1,3-bis(pyrrolidon-1-yl)-butane, and 83,3 % by weight of Kollidon VA 64.

The granulate was fed into a laboratory twin-screw extruder and was extruded at a temperature of 120 °C. No crystalline tramadol hydrochloride could be detected in the clear solidified extrudate by DSC, indicating that the tramadol hydrochloride was present exclusively in a non-crystalline state.

### Example 10

Example 9 was repeated, however the tramadol hydrochloride, 1,3-bis(pyrrolidon-1-yl)-butane, and Kollidon VA 64 were mixed and granulated as such, i. e. without dissolving the active ingredient in the 1,3-bis(pyrrolidon-1-yl)-butane beforehand. No crystalline tramadol hydrochloride could be detected in the clear solidified extrudate by DSC, indicating that the tramadol hydrochloride was present exclusively in a non-crystalline state.

### Example 11 (Comparison)

A homogeneous powdery mixture of 4,2 % by weight of tramadol hydrochloride, and 95,8 % by weight of Kollidon VA 64 was fed into the twin-screw extruder as used in examples 9 and 10. At 120 °C, extrusion failed due to the extremely high torque. No melt could be prepared.

### Example 12 (Comparison)

A homogeneous powdery mixture of 4,2 % by weight of tramadol hydrochloride, and 95,8 % by weight of Kollidon VA 64 was fed into the twin-screw extruder as used in examples 9 and 10. Extrusion was carried out at 125 °C. A turbid melt was obtained (as opposed to the clear melt obtained in examples 9 and 10). The turbidity of the melt indicated that the melt still contained undissolved particles or aggregates.

### Example 13 (Comparison)

A homogeneous powdery mixture of 4,2 % by weight of tramadol hydrochloride and 95,8 % by weight of Kollidon VA 64 was fed into the twin-screw extruder as used in examples 9 and 10. Extrusion was carried out at 140 °C. A turbid melt was obtained. The turbidity of the melt indicated that the melt still contained undissolved particles or aggregates.

## Claims

1. A composition which comprises a solid or semi-solid matrix having at least one active ingredient uniformly dispersed therein, the matrix comprising at least one pharmaceutically acceptable matrix-forming agent selected from pharmaceutically acceptable polymers having a glass transition temperature Tg of from 50° to 180°C; and
1,3-bis(pyrrolidon-1-yl)-butane, wherein the matrix contains less than 6% by weight of water or any compound that is liquid at ambient temperature and has a higher volatility than water, and wherein the solubility of the active ingredient in water at 25 °C is less than 1g/100ml.

2. The composition of claim 1, wherein the matrix comprises, relative to the weight of the matrix, from about 1 to 30 % by weight of 1,3-bis(pyrrolidon-1-yl)-butane and about 50 to 98 % by weight of one or more matrix-forming agents, and from about 1 to 49 % by weight of an active ingredient or a combination of active ingredients.

3. The composition of claim 1 or 2, wherein the active ingredient is dispersed in the matrix in a state of a solid solution.

4. The composition of any one of claims 1 to 3, wherein the pharmaceutically acceptable polymer is selected from the group consisting of homopolymers of N-vinyl lactams,
copolymers of a N-vinyl lactam and one or more comonomers selected from nitrogen-containing monomers and oxygen-containing monomers,
cellulose esters and cellulose ethers,
high molecular polyalkylene oxides,
polyacrylates and polymethacrylates,
oligo- and polysaccharides,
poly(hydroxy acids), or mixtures thereof.

5. The composition of claim 4, wherein the pharmaceutically acceptable polymer is selected from
polyvinylpyrrolidone,
a copolymer of N-vinyl pyrrolidone and a vinyl carboxylate,
a hydroxyalkylcellulose, a hydroxyalkylalkylcellulose,
poly(lactic acid), poly(glyolic acid), poly(3-hydroxybutyrate), poly(3-hydroxyvalerate), poly(3-hydroxybutyrate-co-3-hydroxyvalerate), or mixtures thereof.

6. The composition of claim 5, wherein the pharmaceutically acceptable polymer is selected from a copolymer of N-vinyl pyrrolidone and vinyl acetate and hydroxypropylcellulose.

7. The composition of any one of the preceding claims, wherein the active ingredient is selected from meloxicam and telmisartan.

8. The composition of any one of the preceding claims, wherein the matrix additionally comprises at least one additive selected from from the group consisting of surfactants, flow regulators, disintegrants, bulking agents, lubricants, effervescent agents, colorants, flavourings.

9. A dosage form comprising, or consisting of, the composition of any one of the preceding claims.

10. A method for preparing a composition of claim 1 which comprises:
a) dissolving the active ingredient in a sufficient amount of 1,3-bis(pyrrolidon-1-yl)-butane to obtain an active ingredient solution;
b) providing a powdery composition comprising the matrix-forming agent;
c) blending the active ingredient solution into the powdery composition to obtain a uniform granulate;
d) heating the uniform granulate to an elevated temperature to obtain a melt; and
e) allowing the melt to solidify to obtain a solid dispersion product.

11. A method for preparing a composition of claim 1 which comprises:
a) heating the matrix-forming agent to an elevated temperature to obtain a matrix-forming agent melt;
b) dissolving the active ingredient in a sufficient amount of 1,3-bis(pyrrolidon-1-yl)-butane to obtain an active ingredient solution;
c) adding the active ingredient solution to the matrix-forming agent melt; and
d) allowing the melt to solidify to obtain a solid dispersion product.

12. The method of claim 10 or 11, additionally comprising grinding said solid dispersion product.

13. The method of claim 12, additionally comprising compressing said solid dispersion product into a tablet.

14. The method of claim 13, additionally comprising applying a film-coat to the tablet.

## Patentansprüche

1. Zusammensetzung, umfassend eine feste oder halbfeste Matrix mit wenigstens einem gleichförmig darin dispergierten aktiven Bestandteil, wobei die Matrix wenigstens ein pharmazeutisch akzeptables matrixbildendes Mittel umfasst, das ausgewählt ist unter pharmazeutisch akzeptablen Polymeren mit einer Glasübergangstemperatur Tg von 50°C bis 180°C; und
1,3-Bis(pyrrolidon-1-yl)-butan, wobei die Matrix weniger als 6 Gewichtsprozent Wasser oder einer beliebigen Verbindung umfasst, die bei Umgebungstemperatur flüssig ist und eine höhere Flüchtigkeit als Wasser aufweist, und wobei die Löslichkeit des aktiven Bestandteils in Wasser bei 25°C weniger als 1 g /100 ml beträgt.

2. Zusammensetzung nach Anspruch 1, wobei die Matrix, relativ auf das Gewicht der Matrix bezogen, etwa 1 bis 30 Gewichtsprozent 1,3-Bis(pyrrolidon-1-yl)-butan und etwa 50 bis 98 Gewichtsprozent eines oder mehrerer matrixbildender Mittel, und etwa 1 bis 49 Gewichtsprozent eines aktiven Bestandteils oder einer Kombination aktiver Bestandteile umfasst.

3. Zusammensetzung nach Anspruch 1 oder 2, wobei der aktive Bestandteil in der Matrix im Zustand einer festen Lösung dispergiert ist.

4. Zusammensetzung nach einem der Ansprüche 1 bis 3, wobei das pharmazeutisch akzeptable Polymer ausgewählt ist unter
Homopolymeren von N-Vinyllactamen,
Copolymeren eines N-Vinyllactams und eines oder mehrerer Comonomere, die ausgewählt sind unter Stickstoff enthaltenden Monomeren und Sauerstoff enthaltenden Monomeren,
Zelluloseestern und Zelluloseethern,
hochmolekularen Polyalkylenoxiden,
Polyacrylaten und Polymethacrylaten,
Oligo- und Polysacchariden,
Poly(hydroxysäuren), oder Gemischen davon.

5. Zusammensetzung nach Anspruch 4, wobei das pharmazeutisch akzeptable Polymer ausgewählt ist unter
Polyvinylpyrrolidon,
einem Copolymer von N-Vinylpyrrolidon und einem Vinylcarboxylat,
einer Hydroxyalkylzellulose, einer Hydroxyalkylalkylzellulose,
Poly(milchsäure), Poly(glykolsäure), Poly(3-hydroxybutyrat), Poly(3-hydroxyvalerat), Poly(3-hydroxybutyrat-co-3-hydroxyvalerat), oder Gemischen davon.

6. Zusammensetzung nach Anspruch 5, wobei das pharmazeutisch akzeptable Polymer ausgewählt ist unter einem Copolymer von N-Vinylpyrrolidon und Vinylacetat und Hydroxypropylzellulose.

7. Zusammensetzung nach einem der vorhergehenden Ansprüche, wobei der aktive Bestandteil ausgewählt ist unter Meloxicam und Telmisartan.

8. Zusammensetzung nach einem der vorhergehenden Ansprüche, wobei die Matrix zusätzlich wenigstens einen Zusatzstoff umfasst, der ausgewählt ist unter grenzflächenaktiven Mitteln, Fließregulierungsmitteln, Zerfallshilfsmitteln, Füllstoffen, Gleitmitteln, Schäumungsmitteln, Farbstoffen, Geschmacksstoffen.

9. Dosierungsform, umfassend die oder bestehend aus der Zusammensetzung nach einem der vorhergehenden Ansprüche.

10. Verfahren zur Herstellung einer Zusammensetzung nach Anspruch 1, wobei man:
a) den aktiven Bestandteil in einer ausreichenden Menge 1,3-Bis(pyrrolidon-1-yl)-butan löst, um eine Lösung des aktiven Bestandteils zu erhalten,
b) eine pulverförmige Zusammensetzung bereitstellt, die das matrixbildende Mittel umfasst;
c) die Lösung des aktiven Bestandteils in die pulverförmige Zusammensetzung mischt, um ein gleichförmiges Granulat zu erhalten;
d) das gleichförmige Granulat auf eine erhöhte Temperatur erhitzt, um eine Schmelze zu erhalten; und
e) die Schmelze fest werden lässt, um ein festes Dispersionsprodukt zu erhalten.

11. Verfahren zur Herstellung einer Zusammensetzung nach Anspruch 1, wobei man
a) das matrixbildende Mittel auf eine erhöhte Temperatur erhitzt, um eine Schmelze des matrixbildenden Mittels zu erhalten;
b) den aktiven Bestandteil in einer ausreichenden Menge 1,3-Bis(pyrrolidon-1-yl)-butan löst, um eine Lösung des aktiven Bestandteils zu erhalten;
c) die Lösung des aktiven Bestandteils der Schmelze des matrixbildenden Mittels zugibt; und
d) die Schmelze fest werden lässt, um ein festes Dispersionsprodukt zu erhalten.

12. Verfahren nach Anspruch 10 oder 11, wobei man zusätzlich das feste Dispersionsprodukt mahlt.

13. Verfahren nach Anspruch 12, wobei man zusätzlich das feste Dispersionsprodukt zu einer Tablette verpresst.

14. Verfahren nach Anspruch 13, wobei man zusätzlich die Tablette mit einer Filmbeschichtung versieht.

## Revendications

1. Composition qui comprend une matrice solide ou semi-solide contenant au moins un principe actif uniformément dispersé, la matrice comprenant au moins un agent formant matrice pharmaceutiquement acceptable sélectionné parmi des polymères pharmaceutiquement acceptables ayant une température de transition vitreuse Tg de 50°C à 180°C ; et
du 1,3-bis(pyrrolidon-1-yl)-butane, où la matrice contient moins de 6 % en poids d'eau ou de tout composé qui est liquide à température ambiante et a une volatilité supérieure à l'eau, et où la solubilité du principe actif dans l'eau à 25°C est inférieure à 1 g/100 ml.

2. Composition selon la revendication 1, où la matrice comprend, par rapport au poids de la matrice, d'environ 1 à 30 % en poids de 1,3-bis(pyrrolidon-1-yl)-butane et environ 50 à 98 % en poids d'un ou plusieurs agents formant matrice et d'environ 1 à 49 % en poids d'un principe actif ou d'une association de principes actifs.

3. Composition selon la revendication 1 ou 2, où le principe actif est dispersé dans la matrice à l'état de solution solide.

4. Composition selon l'une quelconque des revendications 1 à 3, où le polymère pharmaceutiquement acceptable est sélectionné dans le groupe constitué de
des homopolymères de N-vinyllactames,
des copolymères d'un N-vinyllactame et d'un ou plusieurs comonomères sélectionnés parmi des monomères contenant de l'azote et des monomères contenant de l'oxygène,
des esters de cellulose et des éthers de cellulose,
des poly(oxyde d'alkylène) de masse moléculaire élevée,
des polyacrylates et des polyméthacrylates,
des oligosaccharides et des polysaccharides,
des poly(hydroxyacides) ou leurs mélanges.

5. Composition selon la revendication 4, où le polymère pharmaceutiquement acceptable est sélectionné parmi
la polyvinylpyrrolidone,
un copolymère de N-vinylpyrrolidone et d'un carboxylate de vinyle,
une hydroxyalkylcellulose, une hydroxyalkylalkylcellulose,
du poly(acide lactique), du poly(acide glycolique), du poly(3-hydroxybutyrate), du poly(3-hydroxyvalérate), du poly(3-hydroxybutyrate-co-3-hydroxyvalérate) ou leurs mélanges.

6. Composition selon la revendication 5, où le polymère pharmaceutiquement acceptable est sélectionné parmi un copolymère de N-vinylpyrrolidone et d'acétate de vinyle et de l'hydroxypropylcellulose.

7. Composition selon une quelconque des revendications précédentes, où le principe actif est sélectionné parmi le méloxicam et le telmisartan.

8. Composition selon l'une quelconque des revendications précédentes, où la matrice comprend additionnellement au moins un additif sélectionné dans le groupe constitué des tensioactifs, des régulateurs de flux, des désintégrants, des agents gonflants, des lubrifiants, des agents effervescents, des colorants, des aromatisants.

9. Forme posologique comprenant ou consistant en la composition selon l'une quelconque des revendications précédentes.

10. Procédé de préparation d'une composition selon la revendication 1, lequel comprend :
a) la dissolution du principe actif dans une quantité suffisante de 1,3-bis(pyrrolidon-1-yl)-butane pour obtenir une solution de principe actif ;
b) la mise à disposition d'une composition de poudre comprenant l'agent formant matrice ;
c) le mélange de la solution de principe actif dans la composition de poudre pour obtenir un granulat uniforme ;
d) le chauffage du granulat uniforme à une température élevée pour obtenir une masse fondue ; et
e) la solidification de la masse fondue pour obtenir un produit de dispersion solide.

11. Procédé de préparation d'une composition selon la revendication 1, lequel comprend :
a) le chauffage de l'agent formant matrice à une température élevée pour obtenir une masse fondue d'agent formant matrice ;
b) la dissolution du principe actif dans une quantité suffisante de 1,3-bis(pyrrolidon-1-yl)-butane pour obtenir une solution de principe actif ;
c) l'addition de la solution de principe actif à la masse fondue d'agent formant matrice ; et
d) la solidification de la masse fondue pour obtenir un produit de dispersion solide.

12. Procédé selon la revendication 10 ou 11, comprenant additionnellement le broyage dudit produit de dispersion solide.

13. Procédé selon la revendication 12, comprenant additionnellement la compression dudit produit de dispersion solide en un comprimé.

14. Procédé selon la revendication 13, comprenant additionnellement l'application d'un enrobage pelliculaire au comprimé.
